(19) Europäisches Patentamt
European Patent Office
Office européen des brevets

(11) Publication number: 0 347 078 A1

# (12) EUROPEAN PATENT APPLICATION

(21) Application number: 89305605.1

(22) Date of filing: 02.06.89

(51) Int. Cl.4: C12N 9/64 , C12N 15/00 , A61K 37/547 , C12N 5/00 , A61K 39/395

(30) Priority: 03.06.88 GB 8813113
28.10.88 GB 8825330

(43) Date of publication of application:
20.12.89 Bulletin 89/51

(84) Designated Contracting States:
AT BE CH DE ES FR GB GR IT LI LU NL SE

(71) Applicant: CELLTECH LIMITED
216 Bath Road
Slough Berkshire SL1 4EN(GB)

(72) Inventor: Emtage, John Spencer
49 Temple Mill Island
Marlow Buckinghamshire Sl7 1SQ(GB)
Inventor: Harris, Timothy John Roy
Field House Beenham
Berkshire RG7 5NX(GB)

(74) Representative: Mercer, Christopher Paul et al
Carpmaels & Ransford 43, Bloomsbury Square
London WC1A 2RA(GB)

## (54) Hybrid proteins.

(57) Recombinant DNA technology is employed to produce a single chain protein in which a Kringle domain and a serine protease domain of a plasminogen activator are linked to the heavy chain variable region of fibrin specific antibody. The single chain protein is associated with a complementary light chain variable domain of an antifibrin antibody so as to form a fibrin binding site. The construct has in vitro and in vivo clot lysis activity, the activity in vivo being similar to that of tPA, while fibrin binding is enhanced relative to tPA.

EP 0 347 078 A1

# HYBRID PROTEINS

## Field of the Invention

The present invention relates to hybrid proteins and in particular to hybrid plasminogen activators. The present invention also relates to the production of such hybrid proteins by recombinant DNA technology and to vectors and host cells for use in such production.

## Background of the Invention

It is well known that the presence of blood clots in the blood vessels of a mammal, particularly a human, can be life threatening and can lead to such acute cardiovascular diseases as infarct (transmural myocardial infarctions), stroke, pulmonary embolism, deep vein thrombosis, peripheral arterial occlusion and other venous thromboses. As the mechanism of the formation and clearance of such blood clots has been elucidated, it has become apparent that there may be a variety of agents which could be of use in preventing or treating such diseases.

A particular class of such agents is the plasminogen activators. These agents act by converting plasminogen to plasmin. The plasmin then acts on the fibrin in blood clots, causing it to break down and the clot to be cleared. There are three well known plasminogen activators, being streptokinase, urokinase and tissue plasminogen activator.

Streptokinase is somewhat different from the other two agents, in that it does not act enzymatically on plasminogen, but causes a conformational change in plasminogen such that it becomes enzymatically active. Among its clinical problems are that it is highly antigenic, being derived from a bacterium, and that it does not bind to fibrin. Thus, its use can lead to the patient's immune system reacting against it and clearing it from the blood before it can effect clearance of a clot. Its use can also lead to non-specific plasminogen activation, causing undesired bleeding. The use of streptokinase therefore has problems in that its side effects militate against its re-use in the same patient.

Urokinase, which is generally now referred to as u-PA (urokinase-type plasminogen activator), was originally isolated from urine. It is nowadays available as a product of recombinant DNA technology. u-PA acts enzymatically on plasminogen to cause its cleavage to plasmin. Since it can be derived from mammalian, for instance human, sources, it is not antigenic when administered to a patient. However, it does not bind to fibrin and thus its use can lead to non-specific plasminogen activation, causing undesired bleeding.

Tissue plasminogen activator (t-PA) was originally isolated from various human tissues in small quantities. Subsequently, it was discovered that certain melanoma cells, in particular Bowes melanoma cells, secreted reasonable amounts of t-PA. More recently, t-PA has become available as a product of recombinant DNA technology. For instance, the cloning of the t-PA gene from the Bowes melanoma cell line was reported by Pennica et al. [1] in 1983. t-PA has the advantages that it is not antigenic and that it has a relatively high affinity for fibrin. It therefore has a lesser tendency to cause non-specific plasminogen activation, thus reducing the amount of undesired bleeding which may occur. It is therefore potentially a better thrombolytic agent than urokinase.

However, in vivo it is cleared very rapidly and therefore cannot be administered by bolus injection. It is thus necessary to administer large doses.

The amino acid sequences of both u-PA and t-PA have now been elucidated. These and the arrangement of the disulphide bridges in the molecules are shown in Figures 1 and 2 respectively of the accompanying drawings. u-PA (Figure 1) as translated from mRNA comprises an N-terminal signal peptide S, followed by a domain which resembles epidermal growth factor E, a Kringle domain K and, at its C-terminal end, a serine protease domain P. The Kringle domain is a highly convoluted, cysteine disulphide linked domain similar to domains in such molecules as plasminogen. After secretion, the signal peptide S is cleaved off to form single chain u-PA, which is subsequently cleaved by plasmin to produce a two chain form of u-PA having the two chains connected by cysteine disulphide links. The so-called A-chain of two chain u-PA comprises the E and K domains and the so-called B-chain comprises the P domain.

t-PA (Figure 2) as translated from mRNA comprises an N-terminal signal peptide S, a leader domain L, a domain similar to a finger of fibronectin F, an epidermal growth factor-like domain E, two Kringle domains K1 and K2 and, at its C-terminal end, a serine protease domain P. After secretion, the S and L regions are cleaved off to form single chain t-PA. This is subsequently cleaved to give two chain t-PA, the A-chain

comprising the F, E, K1 and K2 domains and the B chain comprising the P domain.

A more full description of the structures and functions of u-PA and t-PA is given by Harris [2].

Although t-PA has been shown to have potential as a thrombolytic agent in vivo, it would be desirable to improve both its half-life and its specificity, or to produce a novel molecule having thrombolytic properties, but having improved half-life and specificity as compared to t-PA. In order to achieve this end, it has been proposed that new plasminogen activators could be produced by use of recombinant DNA technology.

One approach to this has been colloquially known as "domain shuffling". It has been proposed that improved plasminogen activators may be produced by deleting domains from existing plasminogen activators or by combining domains from one activator with other domains from another activator. Some of the domain shuffling experiments that have been carried out are set forth by Harris [2]. Similar proposals are also set forth in WO87/03906 (Upjohn).

A second approach, which is aimed at improving the specificity of plasminogen activators, is to link a plasminogen activator, or a domain thereof, to a molecule having high fibrin binding activity. The first reported work in this area described the cross-linking of the serine protease domain (B chain) of u-PA to the A chain of plasminogen (Pln) by partial reduction and oxidation. This formed a plasminogen A chain/u-PA B chain hybrid (Pln A/u-PA B) having the fibrin-binding Kringle domains of plasminogen and the enzymatic activity of u-PA. A 92,000 MW dimer which had a 2-fold lower enzymatic activity but a higher fibrin affinity than u-PA was derived. There was a 5-fold enhancement of plasminogen activation by the hybrid in the presence of soluble fibrin (see Robbins and Tanaka [3]).

Similar studies were carried out using the serine protease domain (B chain) of t-PA. A disulphide-linked dimer (Pln A/t-PA B) was produced (see Robbins and Boreisha [4]). The purified hybrid had some 40% of the enzymatic activity of t-PA and reduced ability to activate plasminogen. It seems likely that the hybrid molecule would not show any enhanced catalytic rate in vivo in the presence of fibrin, and would therefore have reduced clot lysis efficiency.

It has also been proposed to link a plasminogen activator, or a domain thereof, to at least the antigen binding part of an antibody molecule having specificity for fibrin.

For instance, it has been reported that u-PA and t-PA have been chemically cross-linked to such a monoclonal antibody specific for the β chain of fibrin by unidirectional linkers. A similar conjugate comprising an Fab fragment cross-linked to u-PA was also produced (see Bode et al. [5, 6]).

The fibrinolytic activities of these conjugates, u-PA and t-PA were compared using the removal of $_{125}$I-labelled fibrin monomer attached to Sepharose$^R$ beads as an assay for the plasmin produced. The molecules were compared at equivalent enzymatic activities. By this criterion, the antibody conjugates were apparently many fold more active than u-PA or t-PA. Moreover, the increase in activity was inhibited by the heptapeptide to which the antibody had been raised. In in vitro clot lysis assays, the conjugates were 3 to 5-fold more effective than the parent enzymes. In the in vivo rabbit thrombus model, the t-PA conjugate was 2 to 10-fold more efficient than t-PA, suggesting that more potent and selective fibrinolytic agents can be made by this means (see Runge et al. [7, 8]). These conjugates, however, have the disadvantages that they are difficult to make and they are unstable. They may also be readily cleared from a patient's blood stream before they can act in vivo on a blood clot.

A further proposal to this effect is set forth in EP-A-0 187 658 (General Hospital). This application also suggests that the two components may be linked by a peptide-type linkage to form a hybrid wherein the plasminogen activator part is expressed using recombinant DNA technology as a fusion protein with the antibody part.

Another attempt at the recombinant DNA approach to producing antibody/plasminogen activator fusion proteins is described by Schnee et al. [9]. A monoclonal antibody having binding specificity for fibrin was produced. The gene for the variable domain and joining piece sequence of this antibody was inserted into an SV-40 based vector wherein it was joined to a fraction of genomic DNA encoding the first and part of the second constant domains of a mouse antibody constant region. The 3′ end of the antibody gene fraction was joined to a cDNA sequence encoding the B chain of t-PA from the natural cleavage site. The vector thus produced was transfected into a heavy chain loss variant of the myeloma cell line from which the antifibrin monoclonal antibody was derived. Transfected cell lines produced a hybrid molecule comprising an antifibrin variable region, a mouse constant region, including the hinge and part of the second constant domain and, fused to the C-terminal end, the B chain of t-PA. This chain had a molecular weight of about 65,000. It is reported that this chain apparently correctly associated with the light chain secreted by the loss variant myeloma cell line to produce a dimer having a molecular weight of 170,000. The dimer was reported to have been isolated by affinity chromatography.

This hybrid molecule was tested in vitro for fibrin binding and for plasminogen activation. It was found that the hybrid molecule had good fibrin binding and detectable levels of plasminogen activation in vitro.

In a symposium presentation given by Haber [10], one of the co-authors of Schnee et al. [9], results of tests carried out in vivo using the hybrid molecule were given. It appears from these results that the hybrid molecule is not effective as a plasminogen activator in vivo.

The presentation also reported on the production of a second hybrid molecule which is similar to the first molecule, but in which the B chain of t-PA is replaced by the B chain of u-PA. The characterisation of the properties of the second hybrid molecule was not complete.

In the presentation, it was stated that the reason the first hybrid molecule was not very active in vivo is that it cannot generate a free N-terminal residue on the t-PA B chain. It was stated that the intention of the speaker was to produce further hybrid molecules which could be cleaved to produce a free N-terminal residue on the B chain. The presentation also indicated that it will not be necessary to have any of the domains of the plasminogen activator other than the serine protease domain in the hybrid molecule.

## Summary of the Invention

It can be seen from the review above that although much work has been carried out in an attempt to produce improved, novel plasminogen activators, it is still not clear how such improved molecules could be produced.

It is an aim of the present invention to provide a novel plasminogen activator having improved fibrin binding and plasminogen activating properties.

According to the present invention, there is provided a hybrid plasminogen activator comprising:

(1) a single chain protein having:

at its N terminal end the variable domain of the heavy or light chain of a fibrin specific antibody;

a Kringle domain attached to the C terminal end of the variable domain; and

at the C terminal end of the protein, a serine protease domain from a plasminogen activator linked to the C-terminal end of the Kringle domain;

and

(2) a complementary light or heavy chain variable domain of an antifibrin antibody associated with the variable domain in the single chain protein to form an antigen binding site.

The single chain protein and the complementary domain may be associated either non-covalently or preferably covalently, for instance by one or more cysteine disulphide linkages.

Preferably, the antibody heavy and light chain parts are derived from the same antibody, which advantageously is a monoclonal antibody. The monoclonal antibody is conveniently a mouse monoclonal antibody. Advantageously such a mouse monoclonal antibody will have been "humanised", for instance by grafting the mouse hypervariable regions onto a human antibody framework as described by Reichmann et al. [11]. Alternatively, the monoclonal antibody may be a human monoclonal antibody.

It is preferred that the variable domain in the single chain protein is a heavy chain domain and that the complementary domain is a light chain variable domain.

The Kringle domain need not be attached directly to the variable domain. There may be interposed therebetween part or all of one or more antibody constant domains. Preferably, however, there are no plasminogen activator domains between the Kringle domain and the antibody-derived domain(s). For instance, if the variable domain is derived from the antibody light chain, the single chain protein may contain the light chain variable domain attached directly to the Kringle domain.

In a particularly preferred embodiment, the single chain protein contains a heavy chain variable domain, a heavy chain first constant domain and the hinge region or a modified hinge region from the heavy chain of an antibody. Advantageously, the single chain protein does not include any or more than a small part of the second constant domain of the heavy chain. The presence of the hinge region or a modified hinge region in the single chain protein affords an opportunity for the preferred hybrid plasminogen activator of the present invention to form stable dimeric, difunctional structures and will confer flexibility on the hybrid plasminogen activator.

Preferably, the hinge region leads directly into the Kringle domain with no intervening sequences.

The modified hinge region, may, for instance, have a reduced number of cysteine residues to facilitate assembly of the hybrid plasminogen activator into a dimeric structure.

Preferably, where one or more constant domains or parts thereof are present, these are derived from a human antibody. Thus, the hybrid plasminogen activator will be a chimeric antibody of the type described by Neuberger et al. [12, 13].

Preferably, the Kringle domain is attached directly to the serine protease domain such that this part of

the single chain protein closely resembles the C-terminal end of u-PA or t-PA. Less preferably, there may be a short linking sequence between the Kringle and serine protease domains such that these domains are indirectly linked. However, it should be ensured that there are no extensive sequences between the Kringle and serine protease domains.

The Kringle and serine protease domains may have their native sequences. Alternatively, however, the sequences may be varied slightly, for instance to remove any labile sequences, such as the sequence at which cleavage occurs on activation of the plasminogen activator from which the domain is derived. Moreover, the serine protease domain may be a natural or synthetic mutant domain having higher catalytic activity than that of the wild-type domain.

Preferably, the Kringle domain is derived from a plasminogen activator (u-PA or t-PA) and is advantageously the K2 domain of t-PA. Preferably, the serine protease domain is derived from t-PA.

Particular hybrid plasminogen activators according to the present invention will comprise as the single chain protein.

(a) - the variable domain, first constant domain and hinge region of an antifibrin monoclonal antibody heavy chain directly linked to the second Kringle domain and the serine protease domain of t-PA;

(b) - as for (a), except that the hinge region is omitted;

(c) - as for (a), except that the hinge region is altered so that only one cysteine residue is present;

(d), (e) and (f) - as for (a), (b) and (c) respectively, but replacing the t-PA serine protease domain with the u-PA serine protease domain; or

(g) - as for (a), and including the u-PA Kringle domain between the hinge region and the t-PA Kringle domain.

In all the above cases, the single chain protein will be preferably associated with a complete complementary light chain.

According to a second aspect of the present invention, there is provided a hybrid plasminogen activator according to the first aspect of the present invention wherein the single chain protein has been cleaved at the natural cleavage site of the plasminogen activator part of the single chain protein, the serine protease domain remaining bonded, preferably by disulphide linkages, to the remainder of the protein.

Although it will be possible to produce the hybrid plasminogen activator of the present invention by conventional chemical methods, for instance by joining various protein fragments by peptide linkages, it is preferred that it be produced by recombinant DNA technology.

Accordingly, in a third aspect the invention also provides an expression vector including an operon comprising:

a first DNA sequence encoding the variable domain of the heavy or light chain of a fibrin specific antibody;

a second DNA sequence, encoding a Kringle domain of a plasminogen activator, attached in reading frame to the 3′ end of the first DNA sequence; and

a third DNA sequence, encoding a serine protease domain of a plasminogen activator, linked in reading frame to the 3′ end of the second DNA sequence.

The three DNA sequences may independently be cDNA sequences, genomic DNA sequences or mixtures thereof. Preferably, the operons are constructed taking into account the natural intron/exon junctions in the genomic DNA encoding the domains present in the hybrid plasminogen activator. Advantageously, the hybrid plasminogen activators (a) to (g) above are prepared by recombinant DNA technology using predominantly cDNA.

The operon in the expression vector may include further DNA sequences between the first and second DNA sequences, for instance coding for one or more antibody constant domains or parts thereof. Thus the expression vector can be designed so that a host cell transformed with it can express the single chain protein defined according to the first aspect of the invention in any of the forms referred to above.

Preferably, in the expression vector of the present invention, the first DNA sequence encodes a heavy chain variable domain. In this case, it will be possible to transform a host cell which secretes a complementary light chain with the expression vector and obtain expression of the hybrid plasminogen activator of the present invention.

However, in cases where the host cell does not secrete a complementary domain, such as with a non-secreting myeloma line, it will be necessary to endow the host cell with the ability to secrete the complementary domain. This may be done by cotransfecting simultaneously or, preferably, sequentially the host cell with a second expression vector which includes an operon comprising a fourth DNA sequence encoding the complementary variable domain. For instance, a host cell line which secretes a light chain variable domain may be produced by suitable transfection. This may then be further transfected with a vector encoding a complementary heavy chain-based single chain protein.

Alternatively, however, the expression vector of the third aspect of the invention further includes a second operon containing a fourth DNA sequence encoding the complementary variable domain. Advantageously, where the fourth DNA sequence encodes the variable domain of the heavy chain it also encodes at least the first constant domain and the hinge region of a heavy chain. Where the fourth DNA sequence encodes the variable domain of the light chain, advantageously it also encodes the constant domain of a light chain.

The present invention also includes a host cell transfected with an expression vector according to the third aspect of the present invention or, if necessary, cotransfected with the expression vector of the third aspect and second expression vector as defined above.

The host cell may be a prokaryotic or eukaryotic cell, such as a bacterial, yeast or mammalian cell. Preferably, the host cell is a mammalian cell. Most preferably, the host cell is a chinese hamster ovary (CHO) cell, although a myeloid host cell, such as a myeloma or hybridoma cell may alternatively be used. It will also be possible to use other, non-myeloid mammalian cells such as BHK, C127 or L cells. Mammalian cell lines are advantageous in that they have the necessary intracellular mechanisms for processing and assembling such hybrid molecules.

Where the variable domain is derived from the heavy chain of a monoclonal antibody, the host cell may be a heavy chain loss variant hybridoma cell derived from the monoclonal antibody-producing cell.

The recombinant DNA techniques for producing expression vectors are described, for instance, by Maniatis et al. [14]. Processes used to transfect cell lines are shown by Bebbington and Hentschel [28]. Processes used to cultivate host cells and to recover products therefrom are described by Rhodes and Birch [15].

It is envisaged that the hybrid plasminogen activators of the present invention will have advantageous properties as compared to t-PA or the antibody/plasminogen activators described by Schnee or Haber [9 or 10]. The present hybrid plasminogen activators will be very specific for fibrin and bind tightly thereto. Moreover, the plasminogen activating activity of the present hybrid plasminogen activator will be as great as or better than that of t-PA, even *in vivo*. It is surprising that the present hybrid plasminogen activators are efficacious in view of Haber's disclosure that the presence of Kringle domains is *not* required.

It is believed that the improved plasminogen activation is due to the presence of the Kringle region, which transmits a conformational change to the serine protease domain, facilitating the activation of this domain to enable it to activate plasminogen more effectively. The Applicants, however, do not wish to be limited to this explanation of the surprisingly efficacious nature of the present hybrid plasminogen activator.

## Brief Description of the Drawings

An embodiment of the present invention is now described, by way of example only, with reference to the accompanying drawings, in which:

Figure 1 shows the structure and amino acid sequence of u-PA; ▬ indicates inter- and intra-chain disulphide bonds, N-linked oligosaccharide, S signal peptide, E epidermal growth factor-like domain, K Kringle domain, P serine protease domain, cleavage site for activating u-PA, and ● catalytic residues in the serine protease domain;

Figure 2 shows the structure and amino acid sequence of t-PA; ▬ , , S, E, P, , and ● in the figure have meanings equivalent to the same symbols in Figure 1. L indicates the leader peptide, F the fibronectin finger-like domain, K the first Kringle domain, and $K_2$ the second Kringle domain.

Figure 3 shows the structure of plasmid EE7.H3.

Figure 4 shows the structure of plasmid EE7.HcmV.

Figures 5a to 5c show the nucleotide sequence and derived amino acid sequence of the Y22 light chain employed in the hybrid molecule of the present invention. The variable and constant parts of the sequence are indicated.

Figure 6a to 6c show the nucleotide sequence and derived amino acid sequence of the variable portion of the Y22 immunoglobulin heavy chain. Boundaries of the signal peptide, variable domain and CH1 region are indicated.

Figure 7 displays the results of an *in vitro* clot lysis experiment. % clot lysis is plotted against time for an enzyme concentration of 500 U/ml. Enzymes employed were _______ commercially available tPA from Genentech Inc.;

$Y22-K_2SP$ construct of the present invention; and

Kringle 2-serine Protease ($K_2SP$) fragment of tPA.

Figure 8 shows similar in vitro clot lysis results for the $Y22-K_2SP$ construct of the present invention and Genentech tPA at concentrations of 50 U/ml, 100 U/ml and 500 U/ml. ............indicates background.

Modes of carrying out the Invention

Preliminary Experiments

Six vectors were constructed as described below. The first and fourth of these (1 and 2 in Table 1) include DNA encoding hybrid proteins which exemplify those of the present invention. The other four vectors include sequences encoding proteins which lack Kringle domains but are included for purposes of comparison.

pEE7.VCHIH.K2SP.HCMV

Plasmid EE6 is described in WO 89/01036. Plasmid EE7.H3 is shown in Figure 3. It is derived from pEE6 by insertion of the SV40 late promoter into the unique Hind III site of pEE6. This promoter was obtained as a 342 bp Pvu II-Hind III fragment from SV40, containing the origin of replication and Hind III linkers were added to facilitate the insertion. Plasmid EE7.H3 was restricted with HindIII and SmaI and then treated with alkaline phosphatase.

$pSV.V_{NP}\ \gamma_2b\ \Delta$ (CH2, CH3), a plasmid having an operon encoding the heavy chain variable domain, first constant domain and hinge region of an anti-NIP (nitroiodophenyl) antigen (see Figure 1 of Williams and Neuberger [13]), was digested with XhoI and incubated with T4 DNA polymerase to fill in the XhoI site. After removal of the T4 DNA polymerase activity, the DNA was restricted with HindIII and the largest fragment [HindIII-XhoI (blunt)] of approximately 3.8 kb was isolated. This fragment contained the coding sequence for the first constant domain and hinge region of the antibody was inserted between the HindIII and SmaI sites of the restricted and treated EE7.H3 plasmid. This produced the plasmid EE7.CHIH and recreated a unique XhoI site.

$pSV.V_{NP}\ \gamma_2b\ \Delta$ (CH2, CH3) was also digested with NcoI and the synthetic oligonucleotide

5′ CATGGTGGAAGCTTCCAC 3′

was ligated thereto. This reformed the NcoI site with a Kozak consensus sequence (CCACC) around the initiator ATG and placed a HindIII site upstream. Digestion of the ligation product with HindIII allowed the isolation of a HindIII fragment containing the variable domain of the NP gene (approximately 1100bp). This HindIII fragment was cloned into the HindIII site of EE7.CHIH to produce the plasmid EE7.VCHIH.

Plasmid EE7.tPA contains the entire tPA cDNA gene (see Pennica et al. [1]) on a BamHI fragment inserted into the BclI site of plasmid EE7.H3. The BamHI fragment contains most of the 5′ untranslated region of the mRNA and runs to the BclI site in the 3′ untranslated region. The nucleotide sequence of the tPA gene in the plasmid EE7.tPA was changed by site directed mutagenesis to introduce a BglII site immediately in front of the sequence encoding the Asn residue at position 177 (see Figure 2). The sequences encoding the second Kringle and serine protease domains of tPA were then isolated on an XhoII fragment of approximately 1080 bp (from the BglII (XhoII) site at residues 175, 176 to nucleotide 1805 in the 3′ untranslated region of the tPA cDNA).

EE7.VCHIH was digested with XhoI, ligated with the XhoI-BglII adaptor

5′ TCGAGAGATCTC 3′

and then digested with BglII. The XhoII fragment from EE7.tPA containing the sequences encoding the second Kringle and serine protease domains was inserted into this BglII site to produce the plasmid EE7.VCHIH.K2SP.

A PstI fragment (the PstI m-segment) of the human cytomegalovirus (HCMV) dominant immediate early

(IE) gene mRNA was isolated (see Boshart et al. [16]). The fragment spans the promoter region of the gene. A HindIII fragment was generated by ligating synthetic oligonucleotides to the PstI ends to form the following sequence

CTGCAGTCACCGTCCTTGACACGAAGCTT
GACGTCAGTGGCAGGAACTGTGCTTCGAA
PstI HindIII

The thus generated HindIII-fragment was then inserted into the HindIII site of plasmid EE6 to form plasmid EE6.HCMV. (Plasmid EE6 is essentially the same as plasmid EE7.H3 except that the SV40 region is not present.)

The HCMV promoter was isolated on a 2.1 kb HindIII fragment from EE6.HCMV and cloned into EE7.VCHIH.K2SP that had been partially digested with HindIII. A plasmid was isolated that contained the HCMV fragment in the correct orientation and upstream of the V region of the anti-NIP gene. This plasmid in EE7.VCHIH.K2SP.HCMV. The nucleotide sequence across the junction joining the CH2 domain to the second Kringle domain (K2) is:

5' A, GCT, CCT, AAC, CTC, GAG, AGA, TCT, AAC AGT GAC 3'
A P N L E R S N S D

pEE7.VCHI.SPtPA.HCMV

The sequence encoding the major portion of the serine protease domain of t-PA was isolated on a DdeI fragment which starts at nucleotide 1005 and includes the BglII site at nucleotide 2160 (see Harris et al. [17]). To this fragment was ligated the two oligonucleotides:

5' CACCTGCGGCCTGCGCCAGTACAGCCAGCC 3'; and
5'CCGGGTGGACGCCGGACGCGGTCATGTCGGTCGGAGT 3'

These oligonucleotides reconstruct the serine protease domain to the sequence T-C-G-L-R- which starts the serine protease domain (see Figure 2) as defined by intron-exon junctions and places an ApaI site at the 5′ end of the fragment. After ligation and reconstruction, the serine protease domain was isolated on an ApaI-BglII fragment of approximately 1190 bp.

EE7.VCHIH.K2SP was digested with ApaI and BglII. ApaI cut the anti NIP gene at the beginning of the hinge region. Thus, the vector resulting from the above digests lacks the cysteine residues of the $\gamma$ 2b hinge region. The ApaI-BglII fragment encoding the serine protease domain was inserted into this vector to form EE7.VCHI.SPtPA.

EE7.VCHI.SPtPA was digested partially with HindIII and the HCMV promoter (see above) was inserted upstream of the anti-NIP variable region to form EE7.VCHI.SPtPA.HCMV.

The nucleotide sequence across the junction the hinge region and the serine protease domain was determined to be:-

5' GAG, CCC, AGC, GGG, CCC, ACC, TGC, GGC, CTG 3'
E P S G P T263 C G L
Hinge SPtPA

pEE7.VCHIH.SPtPA.HCMV

EE7.VCHIH.K2SP.HCMV was digested with XhoI and the resulting 5′-extensions filled in with T4 DNA polymerase. This fragment was then incubated with SalI and alkaline phosphatase and the resulting large vector fragment isolated.

EE7.VCHI.SPtPA was digested partially with ApaI and the resulting 3′-extensions removed with T4 DNA polymerase. Further digestion with SalI allowed isolation of a fragment of approximately 1800bp with ApaI (blunt) and SalI ends which contained the serine protease domain.

The approximately 1800bp fragment was ligated to the large vector fragment of EE7.VCHIH.K2SP.HCMV to produce EE7.VCHIH.SPtPA.HCMV.

The nucleotide sequence across the junction joining the start of the CH2 domain to the serine protease domain was:

**5' GCT, CCT, AAC, CTC, GAC, ACC, TGC, GGC, CTG, CGC 3'**

**A P N L D T263 C G L R**

**CH2 SPtPA**

## pEE7.VCHI.K2SP.HCMV

EE7.VCHI.SPtPA.HCMV was digested with ApaI and incubated with T4 DNA polymerase to remove the 3′-extensions produced by ApaI. The resulting plasmid was digested with SalI and alkaline phosphatase and the large vector fragment isolated.

EE7.VCHIH.K2SP was digested partially with XhoI, incubated with T4 DNA polymerase to fill in protruding 5′-extensions and then with SalI. A fragment of approximately 1700 bp containing the second Kringle and serine protease domains was isolated. The approximately 1700 bp fragment was ligated to the large vector fragment to produce EE7.VCHI.K2SP.HCMV.

The nucleotide sequence across the junction of the hinge and the start of second Kringle was determined to be:

**5' GAG, CCC, AGC, GTC, GAG, AGA, TCT, AAC, AGT, GAC 3'**

**E P S V E R S N177 S D**

**Hinge K2SP**

## pEE7.VCHIH.SPpUK.HCMV

Prourokinase cDNA is obtained on a fragment running from a HindIII site upstream of the initiator ATG to a StuI site in the 3′ untranslated region. This HindIII-StuI fragment was inserted between the HindIII and SmaI sites of plasmid pSP65. (Plasmid pSP65 is described by Melton et al. [18]).

The majority of the sequence encoding the u-PA serine protease domain can be isolated from pSP65.pUK on a BalI - SacI fragment.

Therefore, pSP65.pUK was digested with BalI and then ligated with the oligonucleotides:

**5' TC, GAG, TTA, AAA, TTT, CAG, TGT, GG 3'; and**

**3' C, AAT, TTT, AAA, GTC, ACA, CC 5'**

This ligation reforms the BalI site, rebuilds the serine protease domain and places an XhoI site immediately 5′ to it. The serine protease domain was then isolated on an XhoI - SacI fragment of approximately 950bp. EE7.VCHIH was digested with SacI and SalI and the 600 bp fragment isolated. EE7.VCHIH.K2SP.HCMV was digested with XhoI and SalI and treated with alkaline phosphatase. The large vector fragment was then isolated. The three fragments described above were ligated together to form

EE7.VCHIH.SPpUK.HCMV.

The nucleotide sequence across the CH2 - SPpUK junction was determined to be:

```
5' GCT, CCT, AAC, CTC, GAG, TTA, AAA, TTT, CAG, TGT 3'
    A    P    N    L    E   L144  K    F    Q    C
              CH2                SPpUK
```

pEE7.VCHI.SPpUK.HCMV

EE7.VCHIH.SPpUK.HCMV was digested with ApaI and XhoI and then incubated with T4 DNA polymerase to produce blunt ends. These ends were joined by ligation to produce EE7.VCHI.SPpUK.HCMV.

The nucleotide sequence across the junction between the start of the hinge and the serine protease domain was determined to be:

```
5' GAG, CCC, AGC, GTC, GAG, TTA, AAA, TTT 3'
    E    P    S    V    E   L144  K    F
   Hinge                         SPpUK
```

Plasmid EE7.preλ.HCmV was constructed by isolating the complete lambda light chain gene (see Wood et al. [19] on a ClaI-BglII fragment and cloning the fragment into plasmid EE7.H3 which had been digested with ClaI and BclI. This produced a plasmid EE7.preλ.

EE7.preλ was digested with HindIII and treated with alkaline phosphatase. The HCMV HindIII fragment (see above) was then inserted to produce the plasmid EE7.preλ.HCMV.

Each of the above six heavy-chain type plasmids was cotransfected into COS cells with plasmid EE7.preλ.HCMV encoding the complete lambda light chain for the anti-NIP antibody. The COS cells were cultured in each of three media and the protein produced by the cells was assayed for anti-NIP activity and for plasminogen activator activity. The results are shown in Table 1, which shows that the molecules retain antigen binding and enzymatic activity.

TABLE 1

| CONSTRUCT | ANTIBODY ACTIVITY A-NIP ($ngml^{-1}$) | PLASMINOGEN ACTIVATOR ACTIVITY ($IUml^{-1}$) | |
|---|---|---|---|
| 1 VCHIH $K_2$SP(t-PA) | | | |
| A | FCS | 250 | 0.96 |
| B | DMEM | 89 | 4.80 |
| C | CT5 | 139 | 10.60 |
| 2 VCHI.$K_2$SP(t-PA) | | | |
| A | FCS | 190 | 0.96 |
| B | DMEM | 147 | 6.70 |
| C | CT5 | 168 | 8.20 |
| 3 VCHIH.SP(t-PA)/ | | | |
| A | FCS | 56 | 3.40 |
| B | DMEM | 61 | 3.40 |
| C | CT5 | 75 | 7.20 |
| 4 VCHI.SP(t-PA) | | | |
| A | FCS | 35 | 0.48 |
| B | DMEM | 49 | 2.40 |
| C | CT5 | 46 | 5.80 |
| 5 VCHIH.SP(pUK) | | | |
| A | FCS | 110 | 21.50* |
| B | DMEM | 86 | 12.40* |
| C | CT5 | 135 | 13.80* |
| 6 VCHI.SP(pUK) | | | |
| A | FCS | 81 | 9.20* |
| B | DMEM | 55 | 6.50* |
| C | CT5 | 89 | 6.50* |

* As measured against a urokinase standard

## Y22 Antibody

Balb/c mice were immunised with a human plasmin-generated fibrinogen degradation product Y. Spleen cell from the immunised mice were fused with P3 x Ag 8653 myeloma cells. A clone (Y22) was isolated that produces monoclonal antibodies of the IgGl k-type. Y22 antibody appears to be directed against a discontinuous, three-dimensional epitope in the D-domain of fibrin. Y22 antibody interferes strongly with the polymerization of fibrin in a thrombin time test. Since a polymerization site is located in the distal end of fibrin, i.e. in the D-domain, this site may be in or near the epitope of Y22. Experiments with $^{99m}$Tc-labelled Y22 in vitro show that Y22 binds rapidly to forming clots. $^{99m}$Tc-Y22 also binds to preformed plasma clots in a plasma milieu, even in the presence of high concentrations of heparin. Clot localization experiments in rabbits and rats show the high fibrin specificity and the potential of $^{99m}$Tc-Y22 for thrombus imaging in vivo.

Polyadenylated RNA was isolated from the Y22 hybridoma cell line using the guanidinium isothiocyanate/lithium chloride method (Cathala et al. [20]). Double stranded cDNA was synthesised (Gubler and Hoffman [21]), and a cDNA library constructed in the plasmid vector CDM8 (Aruffo and Seed [22]),

using BstXI adaptors containing an internal EcoR1 site. A screening probe was synthesised, complementary to the mouse immunoglobulin light chain constant region, this being a 20 mer complementary to residues 4658-4677 of the genomic mouse $C_k$ sequence (Max et al. [23]). The probe was radio-labelled at the 5′ terminus with [$\gamma_{32}$P] ATP using T4 polynucleotide kinase (Amersham International) and used to screen 40,000 colonies from the cDNA library.

Plasmid sequence analysis (Chen and Seeburg [24], modified as described in the Sequenase manual) allowed the identification of a clone which contained the complete leader, variable and constant regions of the light chain, designated pYL22.L12.

The sequence of the leader and variable regions was determined. Examination of the derived amino acid sequence revealed that Y22 has a k light chain of sub-class VII, showing considerable homology with other characterized immunoglobulin genes, thereby enabling the boundaries of the leader, variable and constant domains to be determined. The nucleic acid sequence and derived amino acid sequence for the Y22 light chain are shown in Figs. 5a to 5c.

For expression of the Y22 light chain protein, an EcoRI fragment was isolated from pY22-L12 and inserted into the unique EcoRI site of plasmid EE7.HCMV[28]. The plasmid expressing Y22 light chain was designated pEE7.HCMV.Y22-L1217.

## Cloning of Y22 Heavy Chain

Polydenylated RNA was isolated and converted to double stranded cDNA as described for the light chain cloning. After addition of the BstXI adaptors to the blunt ended molecules, a cDNA library was constructed in pSP64X. pSP64X was derived by the insertion of the XbaI stuffer fragment from CDM8 (Aruffo and Seed [22]) into the XbaI site in the polylinker of pSP64 (Melton et al [18]).

The Y22 anti-fibrin monoclonal antibody is of $\gamma$1 subclass and thus a DNA fragment coding for the constant region of a cloned $\gamma$1 heavy chain was used as a probe for the cloning of the Y22 heavy chain. A 1kb BamHI to EcoR1 restriction fragment comprising the heavy chain constant region of the B72.3 antibody (Whittle et al. [25]) was isolated, $\alpha^{32}$P labelled using the random priming method of Feinberg and Vogelstein [26] and used as to screen the cDNA library. Using this probe a plasmid was isolated from the library and designated pSP64X.Y22H11.

The sequence of the mouse gene has been determined [27]. This shows a BglI site near the end of CH1 and an NdeI site in the hinge region. Analysis of the heavy chain contained in cDNA pSP644X.Y22H11 confirmed their presence and showed that there were no other BglI or NdeI sites in the Y22 heavy chain cDNA. Nucleic acid and derived amino acid sequences for part of the Y22 heavy chain clone are shown in Figs. 6a and 6b.

Thus, the heavy and light chains of the Y22 antibody have been cloned. Using conventional techniques of recombinant DNA technology, the present inventors have been able to replace the anti-NIP variable regions of the constructs referred to above with the anti-fibrin variable region from Y22 heavy chain.

Thus, the following anti-fibrin/plasminogen activator encoding vectors have been constructed.

## Vector comprising a construct with Natural Hinge

### Preliminary Construction:

pSP64X.Y22H11 was digested with NdeI to remove the CH2 and CH3 regions. The codons for the last three amino acids of the hinge and the first five amino acids of CH1 together with restriction sites for XhoI and EcoRI were replaced by ligating oligonucleotides R1271 and R1272.

R1271: 5′-TATGTACGGTACCAGAAGTATCACTCGAG-3′

R1272: 5′-AATTCTCGAGTGATACTTCTGGTACCGTACA-3′

Plasmid EE7. HCMV was constructed as shown in Figure 4. Plasmid EE7.H3 (described above and shown in Figure 3) was digested with HindIII and the HindIII. HCMV fragment was inserted into the digested EE7.H3 The HindIII site between the SV40 region and the HCMV region was then destroyed by partial digestion, blunt ending and religation, followed by selection for the desired deletion.

Following digestion with EcoRI, a Y22 F(ab′) was isolated and inserted into EE7.HCMV [28] that had been digested with EcoRI. These manipulations produced EE7.HCMV.(V.CH1.H)Y22 with a unique XhoI after the codons for the first five amino acids of CH2.

pEE7.HCMV.(V.CH1.H)$_{Y22}$.K2SP

EE7.HCMV.(V.CH1.H)$_{Y22}$ was digested with XhoI and treated with calf intestinal alkaline phosphatase. The XhoI fragment containing the K2SP domains of tPA was then inserted into this vector to produce EE7.HCMV.(V.CH1)$_{Y22}$.K2SP. The nucleotide sequence across the junction of the CH2 region and the K2 domain was determined to be:

```
5'...GTA, CCA, GAA, GTA, TCA, CTC, GAG, AGA, TCT, AAC,
      V    P    E    V    S    L    E    R    S    N
AGT, GAC...3'
 S    D
```

Vectors comprising constructs with Mutated Hinges

Preliminary construction:

To facilitate further work a plasmid containing a unique NruI site was constructed. pSP64X.Y22H11 was digested with BglI, ligated with oligonucleotides R1273 and R1274 and then digested with EcoRI. This allows isolation of an EcoRI fragment containing V.CH1 with NruI and XhoI sites at the start of the hinge region.

R1273: 5′-CGGCCAGCAGCACCAAGGTGGACAAGAAAATTG
TGCCTCGCGATCTCGAG-3′

R1274: 5′ - AATTCTCGAGATCGCGAGGCACAATTTTCTTGT
CCACCTTGGTGCTGCTGGCCGGGT-3′

This EcoRI fragment was cloned into the EcoRI site of EE7.HCMV to produce EE7.HCMV.(V.CH1.NruI)$_{Y22}$.

pEE7.HCMV.(V.CH1.H Δ 2 cys)$_{Y22}$

EE7.HCMV.(V.CH1.NruI)$_{Y22}$ was digested with NruI and ligated with oligonucleotides R1315 and R1316. This reaction mixture was then restricted with XhoI and religated to form EE7.HCMV.(V.CH1.H Δ 2 cys)$_{Y22}$

R1315: 5′ - CGATTGTGGTTGTAAGCCTGCAATAGCTACATG
AC-3′

R1316: 5′ - TCGAGTCATGTAGCTATTGCAGGCTTACAACCA
CAATCG -3′

The nucleotide sequence across the hinge region of this plasmid was determined to be:-

```
... AAA, ATT, GTG, CCT, CGC, GAT, TGT, GGT, TGT, AAG,
     K    I    V    P    R    D    C    G    C    K
CTT, GCA, ATA, GCT, ACA, TGA, CTCGAG...
 P    A*   I    A*   T    stop
```

**A* denote alanines in place of cysteines**

EE7.HCMV.(V.CH1.NruI)$_{Y22}$ was digested with NruI and ligated with oligonucleotides R1317 and R1318. This reaction mixture was then restricted with XhoI and religated to form EE7.HCMV.(V.CH1.H Δ 2 cys)$_{Y22}$F

R1317: 5′- CGATTGTGGTTGTAAGCCTGCAATAGCTACGGT
ACCAGAAGTATCAC -3′

R1318: 5'-TCGAGTGATACTTCTGGTACCGTAGCTATTGCA
GGCTTACAACCACAATCG-3'

pEE7.HCMV.(V.CH1.H Δ 2 $cys)_{Y22}$.K2SP

EE7.HCMV. (V.CH1.H Δ 2 $cys)_{Y22}$.F was digested with XhoI and ligated with the XhoI fragment, containing the kringle 2 and serine protease domains of tPA, to produce EE7.HCMV.(V.CH1.H Δ 2 cys)-$_{Y22}$.K2SP.

pEE7.HCMV.(V.CH1.H Δ 2 $cys)_{Y22}$.KSPpUK

pSP65.pUK was digested with NcoI which cuts at nucleotide 336 in the kringle domain. Synthetic oligonucleotides R1319 and R1320 were ligated to the NcoI site to complete the kringle region up to the intron/exon junction at nucleotide 272 and place an XhoI site upstream of the kringle.

The sequences of the oligonucleotides are as follows:

R1319: 5' - TCGAGATAGATAAGTCAAAAACCTGCT
ATGAGGGGAATGGTCACTTTTACCGAG
GAAAGGCCAGCACTGACAC-3' ;

RL320 : 5' CATGGTGTCAGTGCTGGCCTTTCCTCG
GTAAAAGTGACCATTCCCCTCATAGCA
GGTTTTTGACTTATCTATC - 3'

The KSPpUK domains were then isolated on an XhoI-SacI fragment.

EE7.(VCH1.H)NIP was digested with SacI and SalI and the 600bp fragment isolated. EE7.HCMV.-(VCH1.H Δ 2 $cys)_{Y22}$.F was digested with XhoI and SalI and treated with alkaline phosphatase. The three fragments described above were ligated tcgether to form EE7.HCMV.(V.CH1.H 2 $cys)_{Y22}$.KSPpUK. The nucleotide sequence across the junction was determined to be:

```
5'-GTA, CCA, GAA, GTA, TCA, CTC, GAG, ATA, GAT,
    V    P    E    V    S    L    E    I    D

AAG, TCA, AAA....3'
 K    S    K
```

The present inventors have replaced the anti-NIP light chain with the Y22 light chain and to effect co-transfection of the vectors encoding the heavy and light chain genes.

Thus, the inventors have produced anti-fibrin analogues of the constructs shown in Table 1. As an example, one construct, designated Y22-$K_2$SP was investigated. This construct has a Y22 light chain and a heavy chain comprising the Y22 variable domainand a natural hinge linked to the second Kringle and serine protease domains of tPA. The results presented below indicate that this construct has increased fibrin binding capacity relative to tPA and a similar pattern of enzymatic activity.

Furthermore it will be apparent that dimerisation, through the hinge region, of the constructs, will yield hybrid plasminogen activators of the difunctional dimeric structure referred to above.

EXAMPLE

Expression of the Hybrid Protein Y22-$K_2$SP

CHO cells were transformed with vector pEE7.HCMV.Y22-L1217, encoding the Y22 light chain, and then retransformed with pEE7.HCMV.(V.CH1.H)$_{Y22}$.K2SP. Several clones were then examined to establish their growth and productivity under low serum conditions. Transformed CHO cells which had been previously grown attached to plastic were adapted to grow in suspension in Dulbecco's minimal essential medium (DMEM) containing 1% foetal calf serum (FCS), serum free supplements containing bovine serum albumin, insulin and other such proteins, as contained in commercial serum free media, and 2 x gpt. The cells of each clone were grown in shake flasks and growth and productivity monitored. Productivity was assessed in terms of the plasminogen activating activity of the product hybrid protein (designated Y22-$K_2$SP), as assessed by an S2251 activity assay. This assay measures the plasminogen activating activity of the construct by using it to catalyse the conversion of Lys-plasminogen to plasmin. The plasmin then effects conversion of the chromogenic substrate S2251 to a yellow product and the intensity of colouration is then used as a measure of enzyme activity. Results obtained for 3 CHO cell clones designated RT14, RT11 and RT23 are shown below at Table 2.

TABLE 2

| CLONE | MAX VIABLE CELLS X $10^5$/ml | MAX IU/ml ACCUMULATED | SPECIFIC PRODUCTIVITY IU/$10^6$ CELLS/DAY |
|---|---|---|---|
| RT 14 | 11.83 | 2,245 | 475 |
| RT 11 | 5.50 | 2,000 | 800 |
| RT 23 | 8.80 | 385 | 120 |

RT14 was selected for further study in view of its good growth and product titres. The cell line was shown to be stable in the absence of gpt selection and did not show an apparent decrease in specific productivity with increasing generation number when passaged to 96 generations.

RT14 was suitable for growth on a large scale (1-100l). For example, RT14 was grown up to generation 42 in DMEM + 1% FCS + serum free supplements and transferred to a 1 litre stirred fermenter. A feed of glucose and amino acids was added when the cell density reached 1 x $10^6$ cells/ml and the cells continued to grow to 3 x $10^6$ viable cells/ml. Product was harvested at 50% viability. Productivity data showed a gradual increase of tPA activity early in the fermentation but greater increases after the cells reached maximum viability and entered decline phase. Maximum titre achieved at harvest was ~ 3000IU/ml. This corresponds to a concentration of 6.8$\mu$g/ml assuming a specific activity of 440 IU/$\mu$g. By zymography the fermentation profile showed degradation products which correspond to the size of the serine protease. Formation of these degradation products was markedly reduced by the addition of 50KIU/ml Aprotinin to the fermentation medium.

This process was scaled up to 5L in an airlift fermenter and similar results were obtained. At harvest, product titre was 3,800IU/ml as measured at S2251 activity assay.

Purification of the Protein

Initial purification of the hybrid protein from the fermentations described above was achieved by use of a fibrinogen-Reacti-Gel™ adduct. Fibrinogen-Reacti-Gel™ was prepared from 1,1′-carbonyldiimidazole (CDI) Reacti-Gel™ (6x) from Pierce Chemical Company in the following way.

100mls of CDI-Reacti-Gel™ was filtered through a sintered glass funnel to remove acetone and was washed three times with a coupling buffer (0.1M $Na_2CO_3$ pH6.1). 1g of fibrinogen was dissolved in 200mls of coupling buffer. 1ml of the solution was taken and its $A_{280}$ recorded. The washed Reacti-Gel™ was resuspended in 100mls of the coupling buffer in a 1l roller bottle and the dissolved fibrinogen added. The Gel/ligand mixture was then tumbled at 4°C for 3 days to effect coupling. Afterwards the Gel was washed through a sintered glass funnel with coupling buffer. Excess amines were blocked by the addition to the gel of 300ml of blocking buffer (1M ethanolamine, pH 9.0) and the gel was tumbled overnight, after which supernatant was taken, dialysed against water and the $A_{280}$ measured. By comparison with the $A_{280}$ measured initially a check could be made that all the fibrinogen had coupled to the Reacti-Gel™. Unbound ligand was removed by sequential washes of 1M NaCl, water for injection and coupling buffer. The

fibrinogen-Reacti-Gel adduct was then washed with storage buffer (50mM Tris; 0.1M NaCl; 0.9% Tween 80; pH 8.0) and stored at 4° until needed.

A fibrinogen-Reacti-GelTM column was then packed and culture supernatant loaded onto it. After loading was complete, the column was washed with a sodium phosphate buffer (50mM sodium phosphate buffer; 0.9% NaCl; 0.05% Tween 80; pH 7.4) and then again with the same buffer containing 50mM $\epsilon$-aminocaproic acid to remove any degraded material and intrinsic t-PA produced by the cell line.

The hybrid protein could then be eluted from the column using a buffer of 0.3M glycine hydrochloride, 0.9% NaCl; 0.05% Tween 80 and 50mM $\epsilon$-aminocaproic acid (pH 2.5). Fractions were collected into 1M Tris and the protein eluted in approximately 5 column volumes. A broad peak was observed at $A_{280}$ during elution and fractions collected across this peak were combined and subjected to further purification.

Secondary purification was performed on S-Sepharose. An S-Sepharose column was equilibrated with a buffer of 50mM sodium phosphate and 0.01% Tween 80 (pH 5.5). The eluate from fibrinogen-Reacti-GelTM was diluted two-fold with the same buffer and the pH adjusted to 5.5 with concentrated hydrochloric acid and loaded onto the column. The phosphate buffer was used to wash the column before the hybrid protein was eluted with two column volumes of buffer which was 50mM in sodium phosphate, 0.1M in NaCl and contained 0.01% Tween 80 (pH 7.4).

Fractions collected across the elution peak were assayed using S2288 to locate the most active fractions, and these were combined, concentrated where necessary by ultrafiltration using YM10 membrane and dialysed against an arginine/phosphoric acid/Tween 80 injection buffer (pH 7.0 - 7.5). tPA and the Y22-$K_2$SP construct will both catalyse the conversion of S2288 (Ile-Pro-Arg-paranitroaniline to a yellow product. The intensity of colour generated by the enzyme can then be used to assess enzyme activity.

Electrophoresis by reduced SDS-PAGE revealed bands for the heavy and light chains of the hybrid product as well as three additional bands which are due to clipping of the adduct to generate the B chain of tPA and corresponding Y22 fractions.

Zymograms of the electrophoresed material were also carried out by overlaying a fibrin-agar gel on a developed non-reducing SDS-PAGE gel. Strong clearance of the fibrin agar gel was observed at a single spot indicating that most of the enzymatic activity was associated with a single species identified as the hybrid protein. Some slight clearing of the gel was observed at other positions and this is consistant with some cleavage of the serine protease as mentioned above.

## In Vitro Clot Lysis by the Hybrid Protein

200mls of fresh human blood was attained from a donor and collected into a citrated buffer. 50mls of the blood was decanted while the remaining 150mls was centrifuged at 3000g for 25 minutes in a refrigerated centrifuge. The 50mls of blood was used to form whole blood clots by addition of 2.5ml 0.5M $CaCl_2$, 125 I.U. human thrombin (Sigma) and 0.5-10$\mu$g of $I^{125}$ human fibrinogen (Amersham International). The blood was then aspirated into lengths of silicone tubing closed at each end by 3-way stop cocks and incubated at 37° for 25 minutes.

The plasma from the 150mls of blood was aliquotted into glass scintillation vials and the appropriate concentration of thrombolytic agent under test or buffer was added. In some experiments vials also contained Krebs buffer or buffer or plasma to which 20$\mu$ls of 250 A.T.U./ml Hirudin had been added.

After incubation of the blood in the silicone tubing 1.5-2 cm lengths were cut and the clots poured into plastic petri dishes containing 0.9% NaCl. The clots were washed in saline and then each clot was placed in a plastic tube with 1ml of NaCl for counting in a gamma counter. After counting the saline was decanted and each clot was placed in one of the glass scintillation vials containing human plasma. 25$\mu$l samples of the plasma milieu was removed from each vial at appropriate intervals and counted in the gamma counter. The appearance of $I^{125}$ in the plasma milieu is indicative of clot lysis. The degree of lysis was calculated from the total counts in the clot and the appearance of counts within the plasma over the next 4 to 6 hours.

Using this protocol and fibrinolytic activities of the Y22-$K_2$SP construct of the present invention was compared with that of recombinant tPA as produced by Genentech and the K2SP portion of tPA alone.

Figure 7 shows a comparison between the three enzymes at 500 units/ml in plasma. It is apparent that the construct has comparable clot lysing activity in vitro to that of the other two enzymes.

Figure 8 provides a comparison between Genentech tPA and the construct when the assays are performed in buffer. The graphs indicate that although the activities of both enzymes are comparable, the construct starts to lyse clots more rapidly than tPA itself, that is the construct appears to target the clot.

Active Site Titration of Purified Y22-$K_2$SP Construct

In addition to the clot lysis experiments described above active site titration of the purified construct, of commercially available tPA, and of $K_2$SP alone, was performed in order to determine whether the anti-fibrin, Y22, part of the construct, had an effect on enzyme activity. The intrinsic activities of the three molecules towards the low molecular weight substrate Ile-Pro- Arg-pNA (S2288) and the high molecular weight substrate Lys- plasminogen were determined. In each case activity of the enzyme was measured in terms of the rate of substrate hydrolysis per mole of active enzyme.

The activity of each enzyme was measured against the concentration of an inhibitor, diisopropyl fluorophosphate (DFP) which binds tPA by a 1:1 irreversible reaction. A plot of activity against DFP concentration then yields the activity of the enzyme as the intercept on the ordinate and the concentration of the enzyme as the intercept on the abscissa. The titration was calibrated using commercially available tPA of known concentration as standard. Both standard and construct were converted to their two chain forms by incubation with plasmin before titration.

Table 3 gives the results obtained when each of the enzymes was tested by an S2288 assay.

TABLE 3

| Enzyme | Concentration of Enzyme (nM) | Enzyme Activity AU/min | Specific Activity (AU/min/nM enzyme) | % |
|---|---|---|---|---|
| tPA | 4.6 | 8.3 | 1.80 | 100 |
| $K_2$SP | 3.6 | 9.36 | 2.6 | 144 |
| Y22-$K_2$SP | 4.3 | 8.15 | 1.89 | 105 |

It is apparent from these results that the activity of the construct towards the low molecular weight substrate S2288 is approximately equal to that of tPA and appears to be unaffected by the removal of part of the tPA molecule and addition of the Y22 variable region. The activity of $K_2$SP alone appears to be greater than that of tPA or that of the construct. This suggests that addition of the Y22 variable regions to $K_2$SP decreases activity towards S2288 by about the same amount as does the missing part of the tPA molecule.

The S2251 assay was used to determine the relative activities of each of the enzymes towards their natural substrate plasminogen. Enzyme concentrations as determined in the S2288 titrations were used to compare activities obtained in the absence of DFP.

Table 4 shows the results obtained by active site titration in a S2251 assay.

TABLE 4

| Enzyme | Concentration of Enzyme (nM) | Enzyme Activity AU/min | Specific Activity (AU/min/nM enzyme) | % |
|---|---|---|---|---|
| tPA | 4.6 | 23.8 | 5.17 | 100 |
| $K_2$SP | 3.6 | 20.0 | 5.55 | 107 |
| Y22-$K_2$SP | 4.3 | 7.5 | 1.74 | 34 |

These results demonstrate that tPA and $K_2$SP have similar activity to·.ards the natural substrate, Lys-plasminogen. This implies that removal of the part of the tPA molecule not directly involved in catalysis does not affect its activity. The lower activity of the Y22-$K_2$SP construct indicates that the Y22 portion of the molecule interferes with the reaction utilised in this assay. This may be because of steric hindrance or distortion of the active site structure.

Activity of the Y22-$K_2$SP Construct in vivo

The activity of the Y22-$K_2$SP construct was tested in a rabbit jugular vein model.

Three rabbits were anaesthetised and a catheter was implanted into the femoral artery to allow blood sampling. Uptake of $^{125}I$ by the thyroid gland was prevented by intravenous administration of 0.5ml of a 2% solution of sodium iodide.

An artificial thrombus was made in the jugular vein as follows: the external jugular vein was exposed through a paramedial incision in the neck and carefully dissected. Small side branches were ligated and the facial vein cannulated. A wool thread was introduced into the jugular vein to maintain the clot in place during lysis. Two clamps were positioned at the proximal and distal ends of the jugular vein segment (~ 4cm length) which was then emptied of blood by suction through the facial vein catheter. $^{125}I$-human fibrinogen containing ~ 800,000cpm was mixed with fresh rabbit blood and 10IU thrombin and injected into the jugular vein segment through the facial vein catheter. The clot was aged for 30 minutes before removing both vessel clamps. Thirty minutes after removal of the clamps, administration of the thrombolytic was started.

Thrombolysis was evaluated by monitoring the release of $^{125}I$ into the blood comparing it with the original amount of $^{125}I$ injected and the amount of $^{125}I$ remaining.

The results of preliminary experiments giving a local infusion of the thrombolytic via the ipsilateral ear vein (10ml over a period of 100 minutes) are as shown in Table 5. Lytic activities are compared with those of commercially available tPA.

TABLE 5

| Dose | Rabbit | % Lysis (tPA) | % Lysis (Construct) |
|---|---|---|---|
| 50 000 $IU.Kg^{-1}$ | 1 | 27 | 22 |
| | 2 | 12 | 22 |
| | 3 | 12 | 17 |
| 500 000 $IU.Kg^{-1}$ | 1 | 53 | 54 |
| | 2 | 49 | 25 |
| | 3 | 14 | 13 |

These results indicate that the construct of the present invention is thrombolytically active in vivo and that its activity is broadly similar to that of tPA.

It will be appreciated that the present invention has been described above purely by way of illustration and that variations and modifications of detail may be made without departing from the scope of the invention.

## LIST OF REFERENCES


[1] Pennica et al., Nature, 301, 214-221, 1983.
[2] Harris, Protein Engineering, 1, 6, 449-458, 1987.
[3] Robbins and Tanaka, Biochemistry, 25, 3603-3611, 1986.
[4] Robbins and Boreisha, Biochemistry, 26, 4661-4667, 1987.
[5] Bode et al., Science, 229, 765-767, 1985.
[6] Bode et al., J. Biol. Chem., 262, 10819-10823, 1987.
[7] Runge, PNAS-USA, 84, 7659-7662, 1987.
[8] Runge, Biochemistry, 27, 1153-1157, 1988.
[9] Schnee et al., PNAS-USA, 84, 6904-6908, 1987.
[10] Haber, E., Miami Winter Symposium, 12 February, 1988.
[11] Reichmann et al., Nature, 332, 323-324, 1988.
[12] Neuberger et al., Nature, 312, 604-608, 1984.
[13] Williams and Neuberger, Gene, 43, 319-324, 1986.
[14] Maniatis, T. et al., Molecular Cloning:
A Laboratory Manual, Cold Spring Harbor Laboratory, Cold Spring Harbor, New York, 1982.
[15] Rhodes and Birch, Biotechnology, 6, 518-523, 1988.
[16] Boshart et al., Cell, 41, 521-530, 1985.
[17] Harris et al., Mol. Biol. and Med., 3, 279-292, 1986.

[18] Melton et al., Nuc. Acids. Res., 12, 7035-7070, 1984.
[19] Wood et al., Nature, 314, 446-449, 1985.
[20] Cathala et al., DNA, 2, 239-335, 1983.
[21] Gubler and Hoffman, Gene, 25, 263-269, 1983.
[22] Aruffo and Speed, PNAS-USA, 84, 8573-8577, 1987.
[23] Max et al., J.Biol.Chem., 256, 5116-5120, 1981.
[24] Chen and Seeburg, DNA, 4, 165-170, 1985.
[25] Whittle et al., Prot. Eng., 1, 499-505, 1987.
[26] Feinberg and Vogelstein, Anal. Biochem., 132, 6, 1983.
[27] Honjo et al., Cell, 18, 559-568, 1979
[28] Bebbington and Hentschel, in DNA Cloning, Vol. III, 163-188, ed. D.M. GLOVE, 1987.

## Claims

1. A hybrid plasminogen activator comprising:

(1) a single chain protein having at its N-terminal end the variable domain of the heavy or light chain of a fibrin specific antibody; a Kringle domain attached to the C-terminal end of the variable domain; and at the C-terminal end of the protein, a serine protease domain of a plasminogen activator linked to the C-terminal end of the Kringle domain; and

(2) a complementary light or heavy chain variable domain of an antifibrin antibody associated with the variable domain in the single chain protein to form an antigen binding site.

2. A hybrid plasminogen activator according to Claim 1 wherein the antibody heavy and light chain parts are derived from monoclonal antibody.

3. A hybrid plasminogen activator according to Claim 2 wherein the monoclonal antibody is a non-human antibody which has been humanised by grafting the hypervariable regions of the non-human antibody onto a human antibody framework.

4. A hybrid plasminogen activator according to any one of the preceding claims wherein the variable domain in the single chain protein is a heavy chain domain and the complementary domain is a light chain variable domain.

5. A hybrid plasminogen activator according to any one of the preceding claims wherein there are no plasminogen activator domains between the Kringle domain and the antibody derived domain.

6. A hybrid plasminogen activator according to any one of the preceding claims wherein the single chain protein contains a heavy chain variable domain and a heavy chain first constant domain, but no hinge region.

7. A hybrid plasminogen activator according to Claim 6 wherein the single chain protein further comprises a hinge region or a modified hinge region from the heavy chain of an antifibrin antibody.

8. A hybrid plasminogen activator according to Claim 7 wherein the hinge region leads directly into the Kringle domain with no intervening antibody or plasminogen activator sequences.

9. A hybrid plasminogen activator according to Claim 7 or Claim 8 wherein the hinge has been modified by reduction of the number of cysteine residues.

10. A hybrid plasminogen activator according to any one of Claims 7 to 9 wherein only one cysteine residue is present in the hinge region.

11. A dimeric hybrid plasminogen activator comprising two molecules of a hybrid plasminogen activator according to any one of Claims 7 to 10 linked at their hinge regions.

12. a hybrid plasminogen activator according to any one of Claims 6 to 11 wherein the constant domains are derived from a human antibody.

13. A hybrid plasminogen activator according to any one of the preceding claims wherein the Kringle domain is the K2 domain of tPA.

14. A hybrid plasminogen activator according to any one of the preceding claims wherein the serine protease domain is derived from tPA.

15. A hybrid plasminogen activator according to any one of Claims 1 to 13 wherein the serine protease domain is derived from u-PA.

16. A hybrid plasminogen activator according to any one of the preceding claims wherein the single chain protein has a u-PA Kringle domain included between an antibody heavy chain hinge region and a tPA Kringle domain.

17. A hybrid plasminogen activator according to any one of the preceding claims wherein the single chain protein has been cleaved at the natural cleavage site of the plasminogen activator part of the single chain protein, the serine protease domain remaining bound to the remainder of the protein.

18. A pharmaceutical composition comprising a hybrid plasminogen activator according to any one of the preceding claims and a physiologically acceptable excipient, diluent or carrier.

19. A hybrid plasminogen activator according to any one of Claims 1 to 17 for use as a thrombolytic agent.

20. Use of the hybrid plasminogen activator of any one of Claims 1 to 17 in the manufacture of a thrombolytic agent.

21. An expression vector for production of a hybrid plasminogen activator according to any one of claims 1 to 17, said vector including an operon comprising a first DNA sequence encoding the variable domain of the heavy or light chain of a fibrin specific antibody; a second DNA sequence encoding a Kringle domain of a plasminogen activator attached in reading frame to the 3′ end of the first DNA sequence; and a third DNA sequence, encoding a serine protease domain of a plasminogen activator, linked in reading frame to the 3′ end of the second DNA sequence.

22. An expression vector according to Claim 21 wherein the first DNA sequence encodes a heavy chain variable domain.

23. An expression vector according to Claim 21 or 22 further comprising a fourth DNA sequence encoding the complementary variable domain.

24. A host cell transfected with the expression vector of any one of Claims 21 to 23.

25. A method for the production of a hybrid plasminogen activator comprising transfecting a host cell with the expression vector of any one of Claims 21 to 23 and expressing the encoded domains.

Claims for the following Contracting States: GR, ES

1. A method for the production of a hybrid plasminogen activator comprising expressing:

(1) a single chain protein having at its N-terminal end the variable domain of the heavy or light chain of a fibrin specific antibody; a Kringle domain attached to the C-terminal end of the variable domain; and at the C-terminal end of the protein, a serine protease domain of a plasminogen activator linked to the C-terminal end of the Kringle domain; and

(2) a complementary light or heavy chain variable domain of an antifibrin antibody capable of association with the variable domain in the single chain protein to form an antigen binding site.

2. A method according to Claim 1 wherein the antibody heavy and light chain parts are derived from a monoclonal antibody.

3. A method according to Claim 2 wherein the monoclonal antibody is a non-human antibody which has been humanised by grafting the hypervariable regions of the non-human antibody onto a human antibody framework.

4. A method according to any one of the preceding claims wherein the variable domain in the single chain protein is a heavy chain domain and the complementary domain is a light chain variable domain.

5. A method according to any one of the preceding claims wherein there are no plasminogen activator domains between the Kringle domain and the antibody derived domain.

6. A method according to any one of the preceding claims wherein the single chain protein contains a heavy chain variable domain and a heavy chain first constant domain, but no hinge region.

7. A method according to Claim 6 wherein the single chain protein further comprises a hinge region or a modified hinge region from the heavy chain of an antifibrin antibody.

8. A method according to Claim 7 wherein the hinge region leads directly into the Kringle domain with no intervening antibody or plasminogen activator sequences.

9. A method according to Claim 7 or Claim 8 wherein the hinge has been modified by reduction of the number of cysteine residues.

10. A method according to any one of Claims 7 to 9 wherein only one cysteine residues is present in the hinge region.

11. A method for the production of a dimeric hybrid plasminogen activator comprising linking two molecules of a hybrid plasminogen activator produced by the method of any one of Claims 7 to 10 at their hinge regions.

12. A method according to any one of Claims 6 to 11 wherein the constant domains are derived from a human antibody.

13. A method according to any one of the preceding claims wherein the Kringle domain is the K2 domain of tPA.

14. A method according to any one of the preceding claims wherein the serine protease domain is derived from tPA.

15. A method according to any one of Claims 1 to 13 wherein the serine protease domain is derived from u-PA.

16. A method according to any one of the preceding claims wherein the single chain protein has a u-PA Kringle domain included between an antibody heavy chain hinge region and a tPA Kringle domain.

17. A method according to any one of the preceding claims wherein the single chain protein is cleaved at the natural cleavage site of the plasminogen activator part of the single chain protein, the serine protease domain remaining bound to the remainder of the protein.

18. A method for the production of a pharmaceutical composition comprising mixing a hybrid plasminogen activator as produced by the method of any one of the preceding claims and a physiologically acceptable excipient, diluent or carrier.

19. Use of the hybrid plasminogen activator as produced by the method of any one of Claims 1 to 17 in the manufacture of a thrombolytic agent.

20. A method for the production of an expression vector for production of a hybrid plasminogen activator for use in the method of any one of claims 1 to 17, said method comprising including in a vector an operon comprising a first DNA sequence encoding the variable domain of the heavy or light chain of a fibrin specific antibody; a second DNA sequence encoding a Kringle domain of a plasminogen activator attaching in reading frame to the 3′ end of the first DNA sequence; and a third DNA sequence, encoding a serine protease domain of the plasminogen activator, linked in reading frame to the 3′ end of the second DNA sequence.

21. A method according to Claim 20 wherein the first DNA sequence encodes a heavy chain variable domain.

22. A method according to Claim 20 or 21 wherein a fourth DNA sequence encoding the complementary variable domain is also included in the vector.

23. A method for producing a host cell capable of expressing a hybrid plasminogen activator, the method comprising transfecting the cell with the expression vector as produced by the method of any one of Claims 20 to 22.

24. A method comprising the production of a hybrid plasminogen activator comprising:

(1) a single chain protein having at its N-terminal end the variable domain of the heavy or light chain of a fibrin specific antibody; a Kringle domain attached to the C-terminal end of the variable domain; and at the C-terminal end of the protein, a serine protease domain of a plasminogen activator linked to the C-terminal end of the Kringle domain; and

(2) a complementary light or heavy chain variable domain of an antifibrin antibody associated with the variable domain in the single chain protein to form an antigen binding site.

25. A method comprising the production of an expression vector including an operon comprising a first DNA sequence encoding the variable domain of the heavy or light chain of a fibrin specific antibody; a second DNA sequence encoding a Kringle domain of a plasminogen activator attached in reading frame to the 3′ end of the first DNA sequence; and a third DNA sequence, encoding a serine protease domain of a plasminogen activator, linked in reading frame to the 3′ end of the second DNA sequence.

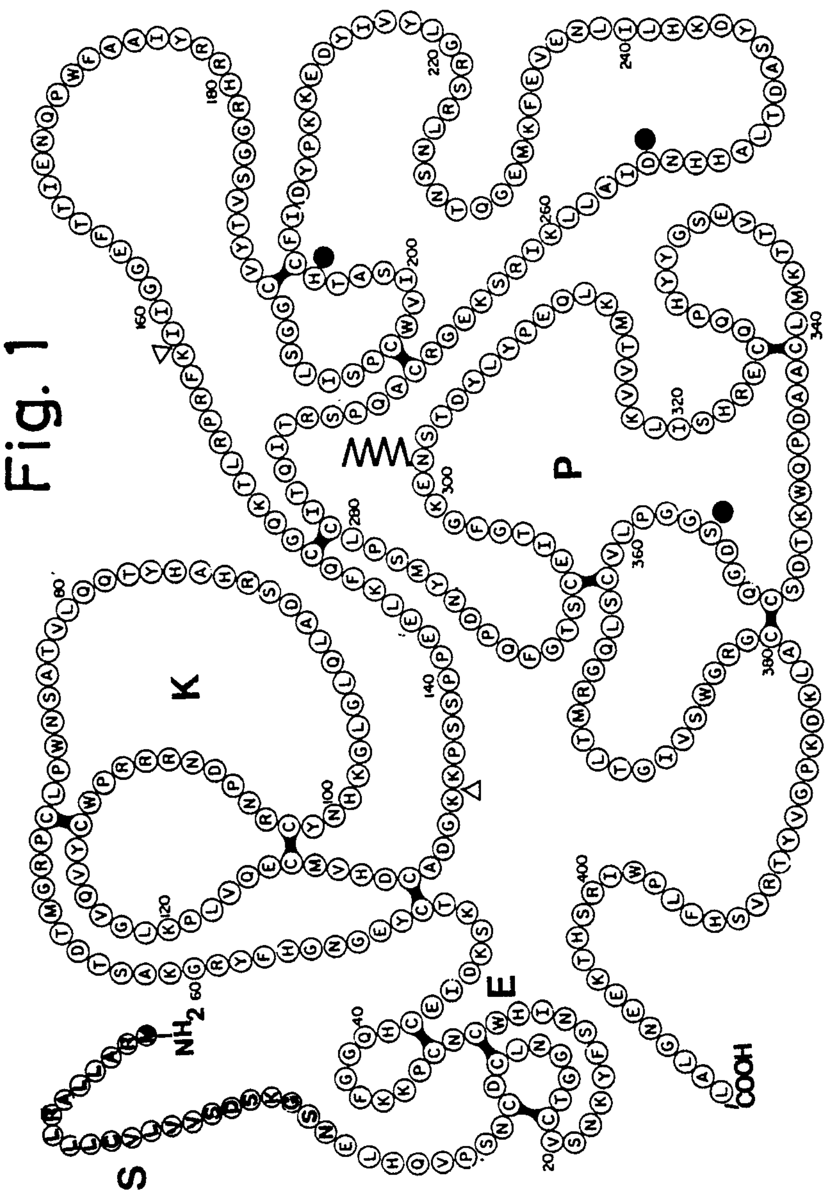


Fig. 1

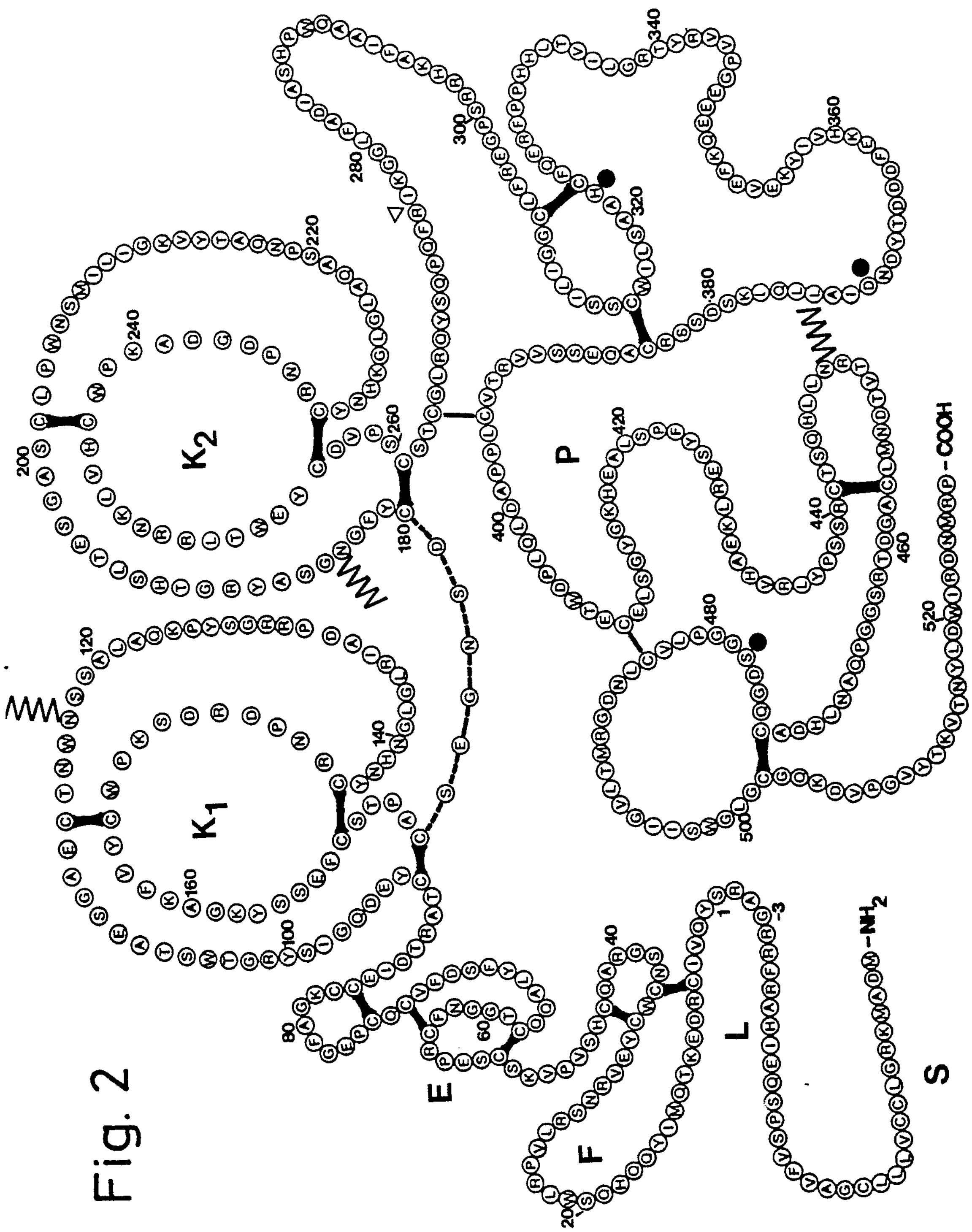


Fig. 2

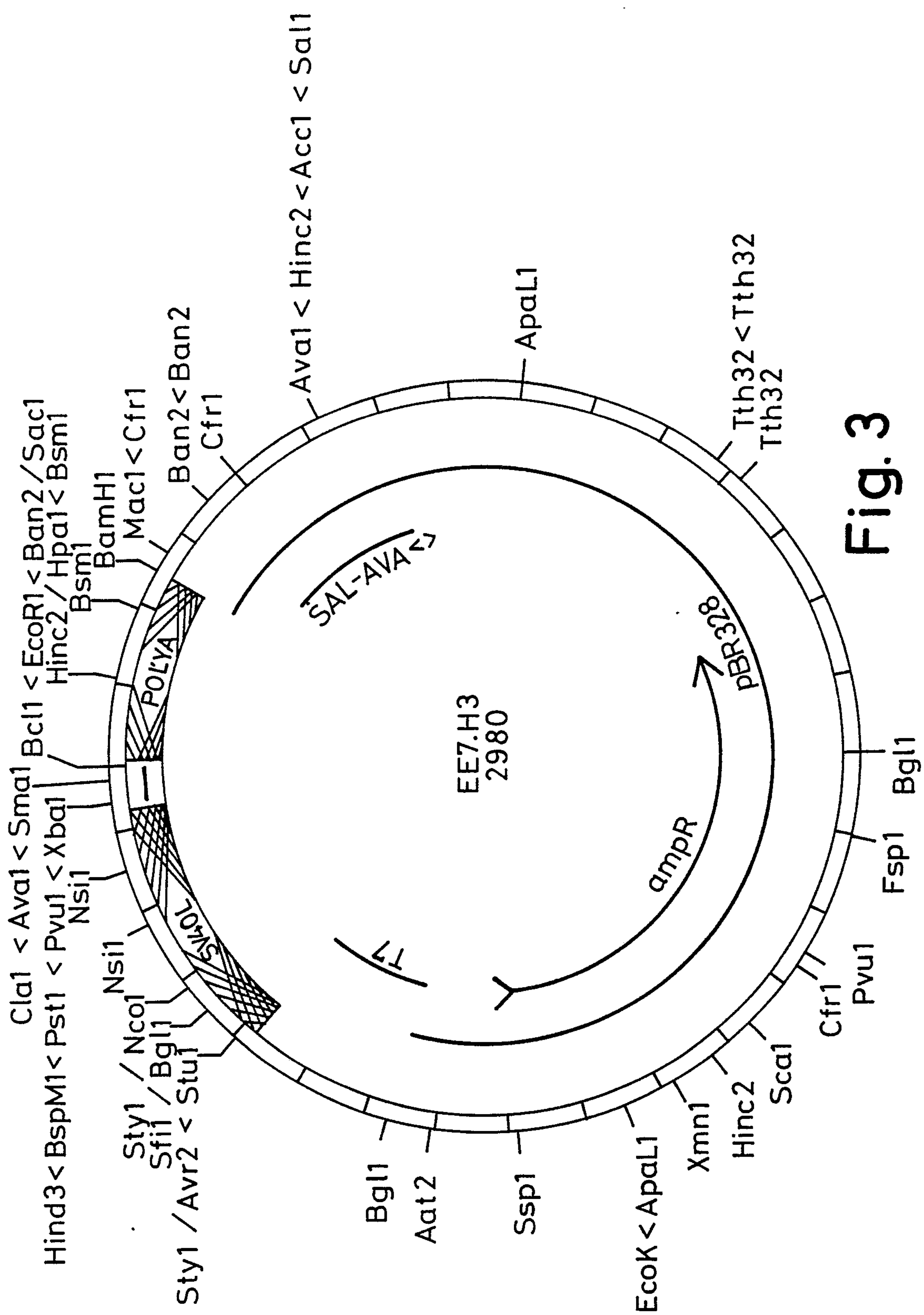
EE7.H3
2980
SAL-AVA
POLYA
SV40L
ampR
pBR328
T7
Ava1 < Hinc2 < Acc1 < Sal1
ApaL1
Tth32 < Tth32
Tth32
Bgl1
Fsp1
Pvu1
Cfr1
Sca1
Hinc2
Xmn1
EcoK < ApaL1
Ssp1
Aat 2
Bgl1
Sty1 / Avr2 < Stu1
Sfi1 / Bgl1
Sty1 / Nco1
Nsi1
Nsi1
Hind3 < BspM1 < Pst1 < Pvu1 < Xba1
Cla1 < Ava1 < Sma1
Bcl1 < EcoR1 < Ban2 / Sac1
Hinc2 / Hpa1 < Bsm1
Bsm1
BamH1
Mac1 < Cfr1
Ban2 < Ban2
Cfr1

Fig. 3

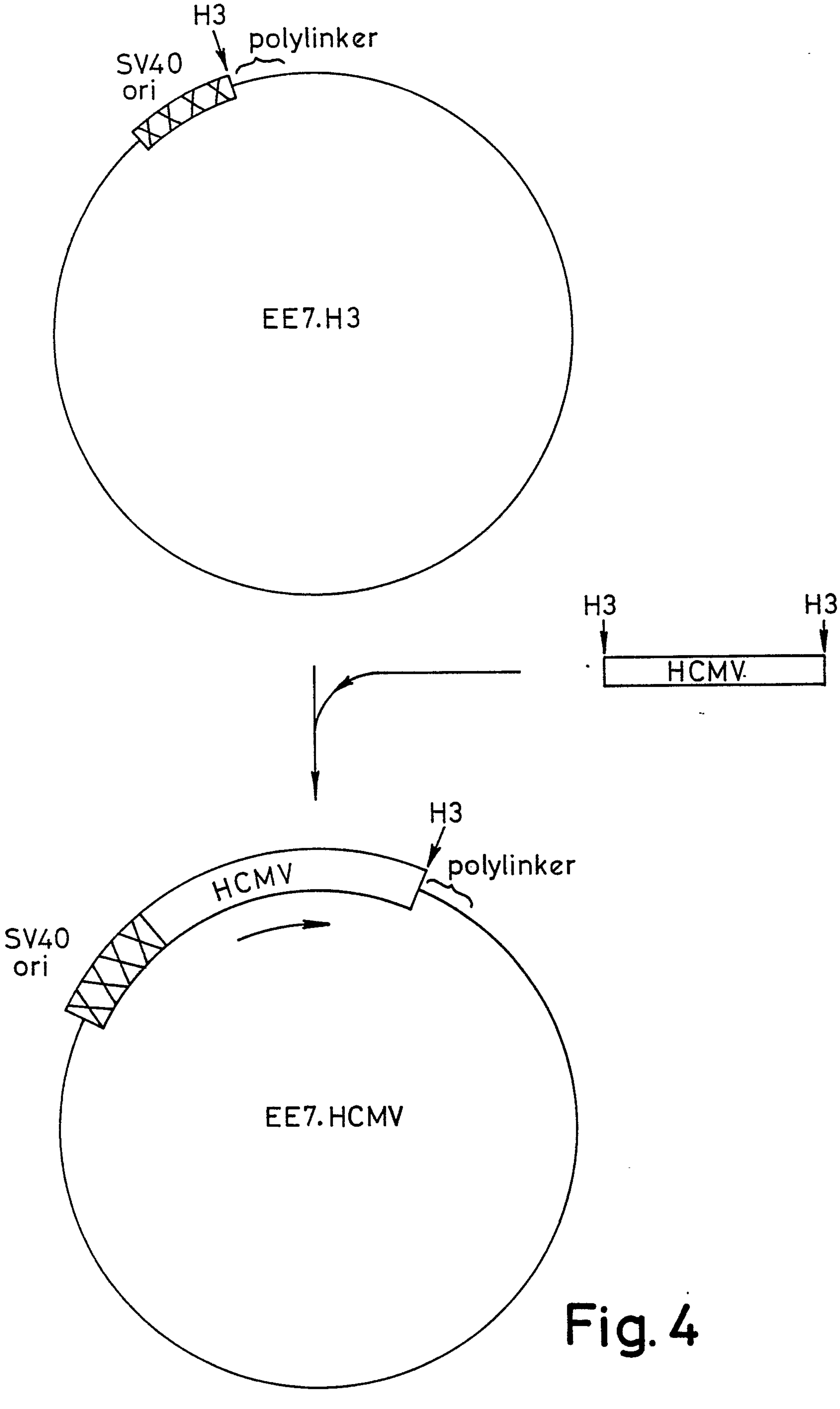
H3
polylinker
SV40
ori
EE7.H3
H3
H3
HCMV
H3
HCMV
polylinker
SV40
ori
EE7.HCMV

Fig. 4

```
     GAATTCAGCATGACCATGCTCTCACTAGCTCCTCTCCTCAGCCTTCTTCTCCTCTGTGTC
1----------------------------------------------------------------- 60
     CTTAAGTCGTACTGGTACGAGAGTGATCGAGGAGAGGAGTCGGAAGAAGAGGAGACACAG

                M  T  M  L  S  L  A  P  L  L  S  L  L  L  L  C  V

     TCTGATTCTAGGACAGAAACAACTGTGACCCAGTCTCCAACATCCCTGTCCGTGGCTACA
61----------------------------------------------------------------120
     AGACTAAGATCCTGTCTTTGTTGACACTGGGTCAGAGGTTGTAGGGACAGGCACCGATGT

     S  D  S  R  T  E  T  T  V  T  Q  S  P  T  S  L  S  V  A  T

     GGAGAGAAAGTCACTATCAGATGCATAACCACCACTGATATTGATGATGATATGAAGTGG
121---------------------------------------------------------------180
     CCTCTCTTTCAGTGATAGTCTACGTATTGGTGGTGACTATAACTACTACTATACTTCACC

     G  E  K  V  T  I  R  C  I  T  T  T  D  I  D  D  D  M  K  W

     TACCAACAGAAGCCAGGGGAACCTCCAAAGCTCCTTATTTCAGAAGGCAATACTCTTCGT
181---------------------------------------------------------------240
     ATGGTTGTCTTCGGTCCCCTTGGAGGTTTCGAGGAATAAAGTCTTCCGTTATGAGAAGCA

     Y  Q  Q  K  P  G  E  P  P  K  L  L  I  S  E  G  N  T  L  R
```

Fig. 5a

```
    CCTGGAGTCCCATCCCGATTCTCCAGCAGTGGCTATGGCTCAGATTTTGTTTTTACAATT
241------------------------------------------------------------300
    GGACCTCAGGGTAGGGCTAAGAGGTCGTCACCGATACCGAGTCTAAAACAAAAATGTTAA

    P  G  V  P  S  R  F  S  S  S  G  Y  G  S  D  F  V  F  T  I


    GAAAACACGCTCTCAGAAGATGTTGCAGATTACTACTGTTTGCAAAGTGATAACATGCCA
301------------------------------------------------------------360
    CTTTTGTGCGAGAGTCTTCTACAACGTCTAATGATGACAAACGTTTCACTATTGTACGGT

    E  N  T  L  S  E  D  V  A  D  Y  Y  C  L  Q  S  D  N  M  P


    TTCACGTTCGGCTCGGGGACAAAATTGGAAATAAGACGGACTGTGGCGGCGCCGTCTGTC
361------------------------------------------------------------420
    AAGTGCAAGCCGAGCCCCTGTTTTAACCTTTATTCTGCCTGACACCGCCGCGGCAGACAG

    F  T  F  G  S  G  T  K  L  E  I  R  R |T  V  A  A  P  S  V
                                          |
                           variable <-----+-----> constant


    TTCATCTTCCCGCCATCTGATGAGCAGTTGAAATCTGGAACTGCCTCTGTTGTGTGCCTG
421------------------------------------------------------------480
    AAGTAGAAGGGCGGTAGACTACTCGTCAACTTTAGACCTTGACGGAGACAACACACGGAC

    F  I  F  P  P  S  D  E  Q  L  K  S  G  T  A  S  V  V  C  L
```

Fig. 5b

```
    CTGAATAACTTCTATCCCAGAGAGGCCAAAGTACAGTGGAAGGTGGATAACGCCCTCCAA
481-------------------------------------------------------------540
    GACTTATTGAAGATAGGGTCTCTCCGGTTTCATGTCACCTTCCACCTATTGCGGGAGGTT

    L  N  N  F  Y  P  R  E  A  K  V  Q  W  K  V  D  N  A  L  Q

    TCGGGTAACTCCCAGGAGAGTGTCACAGAGCAGGACAGCAAGGACAGCACCTACAGCCTC
541-------------------------------------------------------------600
    AGCCCATTGAGGGTCCTCTCACAGTGTCTCGTCCTGTCGTTCCTGTCGTGGATGTCGGAG

    S  G  N  S  Q  E  S  V  T  E  Q  D  S  K  D  S  T  Y  S  L

    AGCAGCACCCTGACGCTGAGCAAAGCAGACTACGAGAAACACAAAGTCTACGCCTGCGAA
601-------------------------------------------------------------660
    TCGTCGTGGGACTGCGACTCGTTTCGTCTGATGCTCTTTGTGTTTCAGATGCGGACGCTT

    S  S  T  L  T  L  S  K  A  D  Y  E  K  H  K  V  Y  A  C  E

    GTCACCCATCAGGGCCTGAGCTCGCCCGTCACAAAGAGCTTCAACAGGGGAGAGTGTTAG
661-------------------------------------------------------------720
    CAGTGGGTAGTCCCGGACTCGAGCGGGCAGTGTTTCTCGAAGTTGTCCCCTCTCACAATC

    V  T  H  Q  G  L  S  S  P  V  T  K  S  F  N  R  G  E  C  *

    AGGGAGAAGTGCCCCCACCTGCTCCTCAGTTCCA
721-----------------------------------754
    TCCCTCTTCACGGGGGTGGACGAGGAGTCAAGGT
```

Fig. 5c

```
     ACCATGAACTTTGTGCTCAGCTTGATTTTCCTTGCCCTCATTTTAAAAGGTGTCCAGTGT
1------------------------------------------------------------60
     TGGTACTTGAAACACGAGTCGAACTAAAAGGAACGGGAGTAAAATTTTCCACAGGTCACA

      M  N  F  V  L  S  L  I  F  L  A  L  I  L  K  G  V  Q  C

                                                 signal <-----

     GAAGTGCAACTGGTGGAGTCTGGGGGACTTAGTGAAGCCTGGAGGGTCCCAGAAACTC
61-----------------------------------------------------------120
     CTTCACGTTGACCACCTCAGACCCCCCTGAATCACTTCGGACCTCCCAGGGTCTTTGAG

     |E  V  Q  L  V  E  S  G  G  D  L  V  K  P  G  G  S  Q  K  L
     |
     |  start of mature protein

     TCCTGTGCAGTCTCTGGATTCACGTTCAGTTCCTATGCCATGTCTTGGGTTCGCCAGATT
121----------------------------------------------------------180
     AGGACACGTCAGAGACCTAAGTGCAAGTCAAGGATACGGTACAGAACCCAAGCGGTCTAA

     S  C  A  V  S  G  F  T  F  S  S  Y  A  M  S  W  V  R  Q  I

     CCGGAGAAGAGGCTGGAGTGGGTCGCAACCATAACTAATGGTGGTGGTTACACCTACTAT
181----------------------------------------------------------240
     GGCCTCTTCTCCGACCTCACCCAGCGTTGGTATTGATTACCACCACCAATGTGGATGATA

     P  E  K  R  L  E  W  V  A  T  I  T  N  G  G  G  Y  T  Y  Y
```

Fig. 6a

```
    CCAGACAGTATGAAGGGTCGATTCACCATCTCCAGAGACAATGCCAAGAACACCCTGTAC
241-------------------------------------------------------------300
    GGTCTGTCATACTTCCCAGCTAAGTGGTAGAGGTCTCTGTTACGGTTCTTGTGGGACATG

    P  D  S  M  K  G  R  F  T  I  S  R  D  N  A  K  N  T  L  Y

    CTGCAAATGGACAGTCTGGGCTCTGAGGACACGGCCATATATTACTGTGCAAGACATCGA
301-------------------------------------------------------------360
    GACGTTTACCTGTCAGACCCGAGACTCCTGTGCCGGTATATAATGACACGTTCTGTAGCT

    L  Q  M  D  S  L  G  S  E  D  T  A  I  Y  Y  C  A  R  H  R

    TATTACGGTAGTAGTTCCTATTGGTACTTCGATGTCTGGGGCGCAGGGACCACGGTCACC
361-------------------------------------------------------------240
    ATAATGCCATCATCAAGGATAACCATGAAGCTACAGACCCCGCGTCCCTGGTGCCAGTGG

    Y  Y  G  S  S  S  Y  W  Y  F  D  V  W  G  A  G  T  T  V  T

    GTCTCCTCACGGTTTTGC
421-------------------438
    CAGAGGAGTGCCAAAACG

    V  S  S| R  F  C
           |
           |start of CH1
```

Fig. 6b

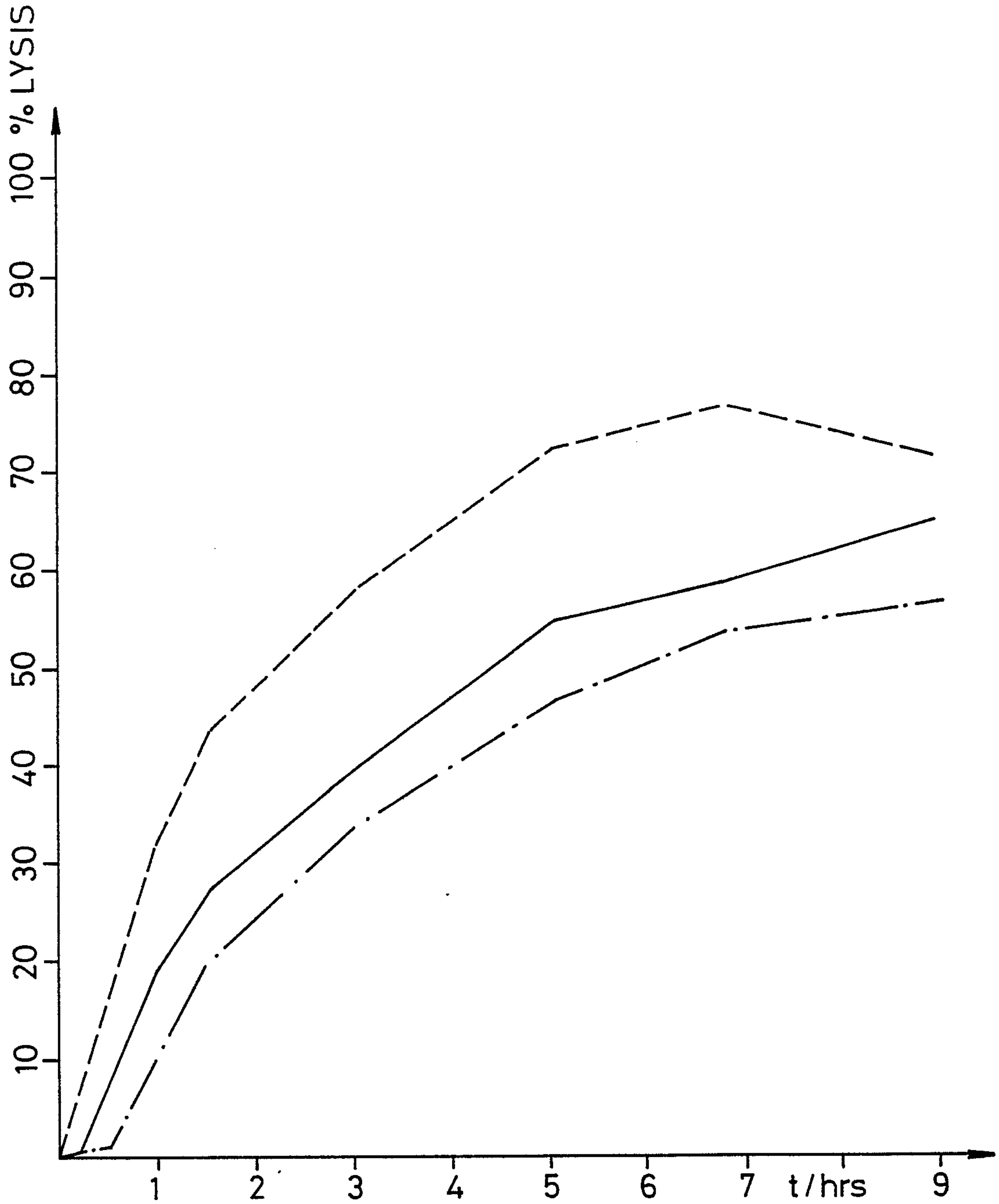


Fig. 7

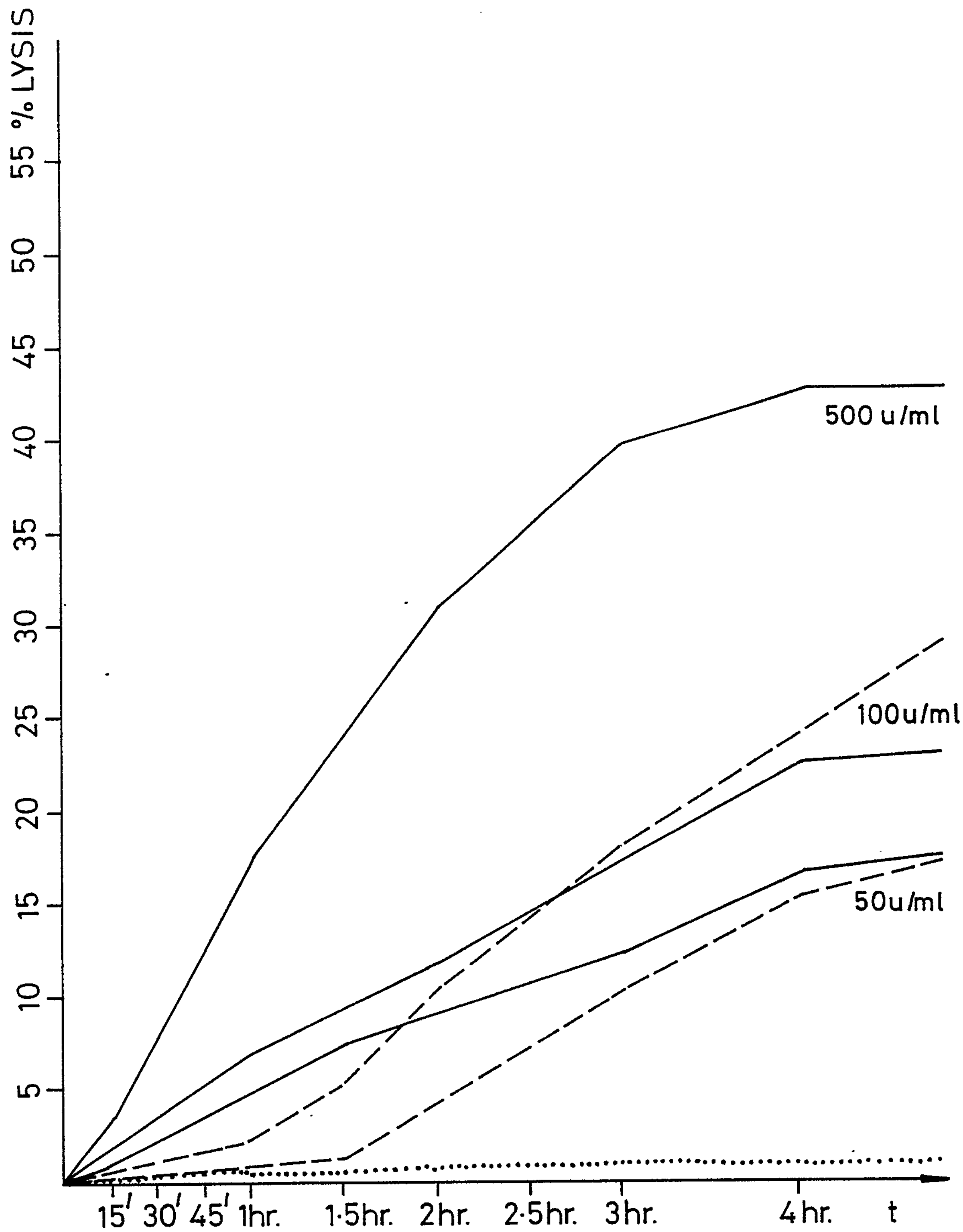
55 % LYSIS
50
45
40
35
30
25
20
15
10
5
500 u/ml
100u/ml
50u/ml
15'
30'
45'
1hr.
1·5hr.
2hr.
2·5hr.
3hr.
4hr.
t

Fig. 8

European Patent Office

# PARTIAL EUROPEAN SEARCH REPORT

which under Rule 45 of the European Patent Convention shall be considered, for the purposes of subsequent proceedings, as the European search report

Application number: EP 89 30 5605

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | PROC. NATL. ACAD. SCI. USA, vol. 84, October 1987, pages 6904-6908, Washington, US; J.M. SCHNEE et al.: "Construction and expression of a recombinant antibody-targeted plasminogen activator"<br>* Whole article *<br>-- | 1,2,4, 14,23-27 | C 12 N 9/64<br>C 12 N 15/00<br>A 61 K 37/547<br>C 12 N 5/00<br>A 61 K 39/395 |
| D,A | PROC. NATL. ACAD. SCI. USA, vol. 84, November 1987, pages 7659-7662, Washington, US; M.S. RUNGE et al.: "Antibody-enhanced thrombolysis: targeting of tissue plasminogen activator in vivo"<br>* Whole article *<br>-- ./. | 1,2,4, 14 | |

TECHNICAL FIELDS SEARCHED (Int. Cl.4)

C 12 N
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-21,23-27

Claims searched incompletely:

Claims not searched: 22

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 14-08-1989 | HUBER-MACK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

European Patent Office

# PARTIAL EUROPEAN SEARCH REPORT

Application number

EP 89 30 5605 -2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| D,A | NATURE, vol.332, March 24,1988, pages 323-327; L. RIECHMANN et al.: "Reshaping human antibodies for therapy" * Page 323, column 2, line 19 - page 325, column 1, line 4 * | 3 | |
| A | WO-A-87 05 934 (CREA et al.) * Page 5, line 23 - page 7, line 5; page 10, line 8 - page 12, line 22; claims * | 1,2,4, 14,23-27 | TECHNICAL FIELDS SEARCHED (Int Cl.4) |
| A | WO-A-88 03 559 (THE GENERAL HOSPITAL CORP.) | | |

-------

EPO Form 1505.3 06.78